(19) Europäisches Patentamt
European Patent Office
Office européen des brevets

(11) Publication number: **0 183 129**
**B1**

# (12) EUROPEAN PATENT SPECIFICATION

(45) Date of publication of patent specification: **09.08.89**

(51) Int. Cl.$^4$: **C 07 D 401/04,** A 61 K 31/47
// C07D215/56

(21) Application number: **85114374.3**

(22) Date of filing: **12.11.85**

## (54) Quinolonecarboxylic acid derivatives.

(30) Priority: **13.11.84 JP 239124/84**

(43) Date of publication of application:
**04.06.86 Bulletin 86/23**

(45) Publication of the grant of the patent:
**09.08.89 Bulletin 89/32**

(84) Designated Contracting States:
**AT BE CH DE FR GB IT LI LU NL SE**

(56) References cited:
**EP-A-0 049 355**
**EP-A-0 106 489**
**EP-A-0 126 355**
**EP-A-0 153 163**
**EP-A-0 167 763**

(73) Proprietor: **KYORIN PHARMACEUTICAL CO., LTD.**
**No. 5, Kanda Surugadai 2-chome**
**Chiyoda-ku Tokyo (JP)**

(72) Inventor: **Irikura, Tsutomu**
**No. 10 28, Ooizumigakuen-cho 7-chome**
**Nerima-ku Tokyo (JP)**
Inventor: **Suzue, Seigo**
**No. 13-4, Aoba 4-chome**
**Kuku-shi Saitama-ken (JP)**
Inventor: **Hirai, Keiji**
**No. 1-2-512, Aoba 1-chome**
**Kuku-shi Saitama-ken (JP)**
Inventor: **Ishizaki, Takayoshi**
**No. 9-6, Sakurada 4-chome Washimiya-machi**
**Kitakatwushika-gun Saitama-ken (JP)**

(74) Representative: **Patentanwälte TER MEER - MÜLLER - STEINMEISTER**
**Mauerkircherstrasse 45**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Detailed Description of the Invention

This invention is concerned with certain novel useful quinolonecarboxylic acid derivatives having antibacterial activities, with a process for their preparation, and with compositions containing them.

Since nalidixic acid which has been employed for treatment of urinary tract infections by gram-negative bacteria, was introduced in 1963, intensive work has been carried out on the further development of quinolonecarboxylic acid analogues.

Thus, recently a remarkable antibacterial activity against not only gram-negative but also gram-positive bacteria occurs for some compounds (e.g. norfloxacin). However, their activity against gram-positive bacteria is fairly less than that against gram-negative bacteria.

Just recently, drugs which have relatively strong activity against gram-positive bacteria have been developed, but shown to possess weaker activity against gram-negative bacteria than the prior compounds (e.g. norfloxacin, ciprofloxacin).

Substituted quinolonecarboxylic acids having antibacterial activity are described in EP—A—0 126 355 and EP—A—0 167 763. However, the compounds of EP—A—0 126 355 bear fluorine atoms at C-6 and C-8 whereas the compounds of EP—A—0 167 763 are substituted at C-6 and C-8 with fluorine or chlorine atoms with the exception that a fluorine substitution is not possible at both positions.

As a result of the investigation, the present inventors have nów unexpectedly found that new derivatives of quinolonecarboxylic acid have excitingly potential activity against gram-positive bactera without decrease of activity against gram-negative bacteria in comparison with prior art analogues and therefore are superior with respect to commercial preparations and investigational drugs in the in vitro and in vivo antibacterial activity against both gram-negative and gram-positive bacteria.

The invention relates to compounds of the formula (I)

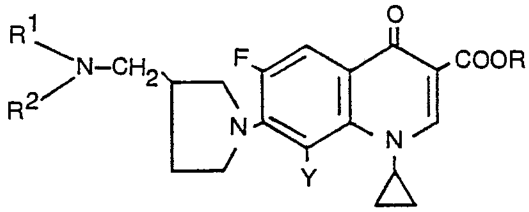

(I)

wherein R is a hydrogen atom or an ethyl group and $R^1$ and $R^2$ are each independently a hydrogen atom, a methyl, an ethyl, a n-propyl or an i-propyl group and Y is a chlorine atom or a bromine atom; the hydrates or the pharmaceutically acceptable acid addition or alkali salts thereof.

The inventive compounds are valuable in human and veterinary medicine because of their greater activity and broader spectrum against both aerobic and anaerobic bacteria.

While the compounds of the present invention show such strong activities against bacteria, their toxicity against mammalian cells is very weak.

The present compounds are well absorbed and distributed into the tissue when administered orally to animals.

The present compounds, therefore, are active at low doses against both gram-positive and gram-negative bacteria and thus constitute valuable agents for the treatment of infectious human, animal or plant diseases.

The compounds of the formula (I) are synthesized by reacting a compound of the formula (II),

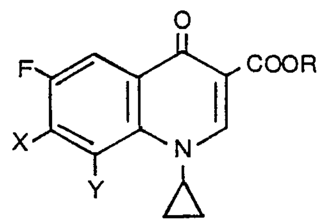

(II)

wherein X is a halogen atom, R and Y are the same as defined above, with a compound of the formula (III),

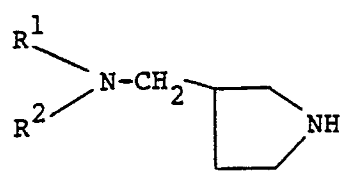

(III)

wherein $R^1$ and $R^2$ are the same as defined above. The reaction is preferably carried out by heating the two reactants in a solvent such as water, alcohols, acetonitrile, dimethylformamide (DMF), dimethyl sulfoxide

(DMSO), hexamethylphosphoric triamide, pyridine and picoline or in absence of the solvent with, if necessary, an acid acceptor such as an inorganic or organic acceptor, e.g. an alkali metal carbonate such as potassium carbonate or *tert*-amine, such as triethylamine, diazabicyclo base at room temperature to 200°C, preferably room temperature to 160°C, more preferably room temperature to 120°C for 1 to several hours. It is desirable that a slight excess (1 to 5 moles) of the secondary amine of the formula (III) is used per mole of the compound of the formula (II) and a solvent is used such that the mixture remains homogeneous after dissolution of the compound of the formula (II) (2 to 10-fold volume per volume of the compound of the formula (III)).

When R in the formula (II) is an ethyl group, the reaction product (carboxylic ester) is hydrolyzed to the corresponding carboxylic acid by the usual manner.

The hydrolysis is carried out by treating the compound of formula (I:R is an ethyl group) with an alkali metal hydroxide solution such as sodium hydroxide, potassium hydroxide, or a mineral acid such as hydrochloric acid, sulfuric acid in water, aqueous alcohols or an appropriate solvent.

Furthermore, the compounds of the formula (I) can be converted, if desired, to the pharmaceutically acceptable salts by the treatment with an acid or an alkali. The acid may be organic or inorganic acids such as, for example, hydrochloric acid, sulfuric acid, phosphoric acid, methanesulfonic acid, acetic acid, oxalic acid and lactic acid. The alkali salts may be, for example, sodium, potassium, magnesium, calcium, aluminum, cerium, chromium, cobalt, copper, iron, zinc, platinum and silver salts.

The compound of the formula (I), the hydrates and salts thereof may be used as medicines in the conventional form of pharmaceutical preparations, which may be, for example, tablets, capsules, powder, ointments, suppositories, injections or eye drops, suitable for peroral, parenteral, enteral or local adminstration.

The following examples will further illustrate the invention without, however, limiting it thereto.

Example 1

8-Chloro-1-cyclopropyl-6-fluoro-1,4-dihydro-7-(3-methylaminomethyl-1-pyrrolidinyl)-4-oxo-3-quinolinecarboxylic acid hydrochloride

A mixture of 8-chloro-1-cyclopropyl-6,7-difluoro-1,4-dihydro-4-oxo-3-quinolinecarboxylic acid (0.4 g), 3-methylaminomethylpyrrolidine (0.17 g) and DBU (0.2 g) in acetonitrile (4 ml) was refluxed for 2 hours and allowed to stand overnight during which time a crystal separated. The crystal, free form of the title compound, was collected by filtration and weighed 0.5 g. The product was dissolved in a mixture of concentrated hydrochloric acid and methanol. Removal of the solvent left the title compound which was recrystallized from ethanol to give yellow prisms (0.3 g), mp 206—120°C (decompd.).

Analysis (%) for $C_{19}H_{21}ClFN_3O_3 \cdot HCl \cdot 1/3\ H_2O$, Calcd. (Found): C, 52.30 (52.05); H, 5.24 (5.03); N, 9.63 (9.45).

In this example, the starting material is also novel and it is synthesized by following process.

Preparation Example of Starting Material 1

N-(3-Chloro-4-fluorophenyl)acetamide

To 3-chloro-4-fluoroaniline (100 g) was slowly added acetic anhydride (200 ml). After allowed to stand for 30 minutes, the reaction mixture was poured into water (1 liter). The resulting precipitate was collected by filtration and recrystallized from aqueous ethanol to give the title compound (119.4 g), mp 118—119°C.

Preparation Example of Starting Material 2

N-(3-Chloro-4-fluoro-6-nitrophenyl)acetamide

To a solution of N-(3-chloro-4-fluorophenyl)acetamide (55 g) in concentrated sulfuric acid (165 ml) was added dropwise concentrated nitric acid (d 1.42, 154 ml) at 5—10°C during an hour with stirring in an ice-salt bath. After stirring for an hour at the same temperature, the reaction mixture was poured into ice water. The resulting precipitate was collected by filtration, sufficiently washed with water and recrystallized from acetonitrile to give the title compound (48.9 g) as yellow needles, mp 114—115°C.

Analysis (%) for $C_8H_6ClFN_2O_3$, Calcd. (Found): C, 41.31 (41.48); H, 2.60 (2.52); N, 12.04 (12.13).

Preparation Example 3

3-Chloro-4-fluoro-6-nitroaniline

A solution of N-(3-chloro-4-fluoro-6-nitrophenyl)acetamide (30 g) in concentrated hydrochloric acid (50 ml) and ethanol (200 ml) was refluxed for 2.5 hours. To the reaction mixture was added ice water (300 ml) and the resulting precipitate was collected by filtration, washed with water and dried to give the title compound (24.9 g) as yellow needles, mp 149.5—150°C.

Analysis (%) for $C_6H_4ClFN_2O_2$, Calcd. (Found): C, 37.82 (37.85); H, 2.11 (2.03); N, 14.70 (14.80).

Preparation Example 4

2,3-Dichloro-4-fluoro-6-nitroaniline

Into a solution of 3-chloro-4-fluoro-6-nitroaniline (14.3 g) in acetic acid (150 ml) was bubbled chlorine gas at 18—20°C during 70 minutes. The reaction mixture was poured into ice water (300 ml) and the

resulting precipitate was collected by filtration, washed with water and recrystallized from ethanol to give the title compound (14.33 g) as yellow needles, mp 161°C.

Analysis (%) for $C_6H_3Cl_2FN_2O_2$, Calcd. (Found): C, 32.03 (32.17); H, 1.34 (1.26); N, 12.45 (12.65).

Preparation Example 5

2,3,4-Trichloro-5-fluoronitrobenzene

To a mixture of anhydrous cupric chloride (13.58 g) and *tert*-butylnitrite (12.4 g) in anhydrous acetonitrile (100 ml) was added portionwise 2,3-dichloro-4-fluoro-6-nitroaniline (18.05 g) at 60—62°C during 30 minutes. After stirring for 30 minutes at 60—65°C, the reaction mixture was poured into chilled 10% diluted hydrochloric acid (300 ml) and extracted with benzene. The organic layer was successively washed with diluted hydrochloric acid and water, dried over anhydrous sodium sulfate and concentrated. The resulting residue was purified by distillation to give the title compound (17.26 g), bp 137—142°C/36 mbar.

NMR (δ in $CDCl_3$), 7.65 (d, J=7.5Hz)

Preparation Example 6

3-Chloro-2,4,5-trifluoronitrobenzene

To a suspension of potassium fluoride (64.9 g) in anhydrous dimethyl sulfoxide (230 ml) was added 2,3,4-trichloro-5-fluoronitrobenzene (54.4 g) at 140°C and stirred at the same temperature for 10 minutes. The reaction mixture was poured into ice water (700 ml) and extracted with petroleum ether. The organic layer was successively washed with water, aqueous potassium carbonate solution and then water, dried over anhydrous sodium sulfate and concentrated. The resulting residue was distilled to give the title compound (9.7 g), bp 95—108°C/40 mbar.

NMR (δ in $CDCl_3$), 7.94 (ddd, J=6.7, 7.6, 9.0Hz)

Preparation Example 7

3-Chloro-2-cyclopropylamino-4,5-difluoronitrobenzene

A solution of cyclopropylamine (2.8 g) and triethylamine (5.1 g) in anhydrous toluene (20 ml) was added dropwise to a solution of 3-chloro-2,4,5-trifluoronitrobenzene (9.7 g) in anhydrous toluene (30 ml) at 3—5°C during 40 minutes with stirring. After stirring for 3 hours at the same temperature, the reaction mixture was poured into ice water (150 ml) and extracted with dichloromethane. The organic layer was washed with water, dried over anhydrous sodium sulfate and then concentrated. The resulting residue was purified by silica gel chromatography using n-hexane-dichloromethane as an eluent to give the title compound (4.4 g) as red oil.

NMR (δ in $CDCl_3$),

0.5—1.0 (4H, m, 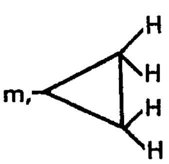 ), 3.0—3.2 (1H, m, 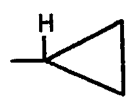 ),

7.19 (1H, s, NH), 7.85 (1H, dd, J=8.2, 9.9Hz, 5—H).

Preparation Example 8

N-(2-Chloro-3,4-difluoro-6-nitrophenyl)-N-cyclopropylacetamide

To 3-chloro-2-cyclopropylamino-4,5-difluoronitrobenzene (4.4 g) was added acetic anhydride (15 ml) with one portion and the mixture was stirred for 30 minutes at room temperature, and then poured into ice water (100 ml). Excessive acetic anhydride was decomposed by potassium carbonate powder, and then the mixture was allowed to stand overnight at 5°C.

The resulting precipitate was collected by filtration and recrystallized from ethyl acetate-n-hexane to give the title compound (2.7 g), mp 98—99.5°C.

Analysis (%) for $C_{11}H_9ClF_2N_2O_3$, Calcd. (Found): C, 45.46 (45.56); H, 3.12 (3.00); N, 9.64 (9.69).

Preparation Example 9

N-(2-Chloro-3,4-difluorophenyl)-N-cyclopropylacetamide

A mixture of N-(2-chloro-3,4-difluoro-6-nitrophenyl)-N-cyclopropylacetamide (2.7 g) and 10% palladium-charcoal (0.5 g) in ethanol (50 ml) was hydrogenated for 40 minutes at 2—3°C under atmospheric pressure in an ice bath. The catalyst was filtered off and the filtrate was concentrated. Then, the resulting crystalline residue was dried in vacuo at room temperature for 10 hours. A solution of the residue in anhydrous dimethylformamide (15 ml) was added dropwise to a solution of *tert*-butyl nitrite (1.72 g) in anhydrous dimethylformamide (10 ml) at 50—52°C during 13 minutes. The reaction mixture was stirred at the same temperature for 5 minutes, then poured into ice water and extracted with ether. The organic layer was successively washed with water, diluted hydrochloric acid and water, dried over anhydrous sodium sulfate and concentrated. The resulting residue was purified by silica gel chromatography using n-hexane-

ethyl acetate as an eluent and recrystallized from petroleum ether to give the title compound (0.44 g), mp 60.5—61.5°C.

Analysis (%) for $C_{11}H_{10}ClF_2NO$, Calcd. (Found): C, 53.78 (53.87); H, 4.10 (4.02); N, 5.70 (5.78).

Preparation Example 10

N-Cyclopropyl-2-chloro-3,4-dfluoroaniline

A solution of N-(2-chloro-3,4-difluorophenyl)-N-cyclopropylacetamide (0.44 g) in 20% diluted hydrochloric acid (7 ml) was heated at 80—100°C for 6 hours with stirring, and then cooled. The reaction mixture was poured into ice water, alkalized with aqueous sodium hydroxide and extracted with ether. The ether layer was washed with water, dried over anhydrous sodium sulfate and then concentrated. The resulting residue was purified by silica gel chromatography using petroleum ether as an eluent to give the title compound (100 mg) as orange oil.

Preparation Example 11

Ethyl 8-chloro-1-cyclopropyl-6,7-difluoro-1,4-dihydro-4-oxo-3-quinolinecarboxylate

A mixture of N-cyclopropyl-2-chloro-3,4-difluoroaniline (100 mg) and diethyl ethoxymethylene-malonate (100 mg) was stirred for 10.5 hours at 100—135°C with removal of generated ethanol by flowing nitrogen gas, and then cooled. Polyphosphoric acid (1 g) was mixed with this, and the mixture was stirred for 3 hours at 125—135°C. After cooling, the reaction mixture poured into ice water, extracted with chloroform. The organic lsyer was successively washed with aqueous potassium carbonate and water, dried over anhydrous sodium sulfate and then concentrated. The resulting residue was purified with silica gel thin layer chromatography using ether as an eluent to give the title compound (11 mg) as colorless needles, mp 160—162.5°C.

NMR (δ in $CDCl_3$),

1.0—1.5 (4H, m, 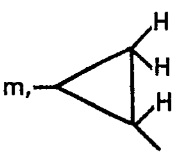 ) 1.40 (3H, t, J=7.0Hz, —$CH_3$), 4.1—4.4 (1H, m, 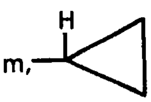 ),

4.38 (2H, q, J=7.0Hz, —$CH_2$—$CH_3$), 8.22 (1H, dd, J=8.8, 9.7Hz, 5—H), 8.66 (1H, s, 2—H)

Preparation Example 12

2,3,4-Trichloro-5-fluoroaniline

To a suspension of iron powder (54.6 g) in water (60 ml), with vigorous stirring at 50—60°C, was slowly added concentrated hydrochloric acid (6.7 ml). After ethanol (150 ml) was mixed 2,3,4-trichloro-5-fluoro-nitrobenzene (75.1 g) was added portionwise to the suspension at 60—70°C during an hour. After stirring for an hour at 80°C, the hot reaction mixture was filtered and the insoluble material was successively washed with hot ethanol (100 ml) and benzene (300 ml). The filtrate and washings were combined and mixed with ice water. The resulting organic layer was collected and the water layer was extracted with benzene (200 ml). The organic layers were washed with water, dried over anhydrous sodium sulfate and then concentrated. The resulting residue was recrystallized from n-hexane to give the title compound (58.6 g) as light brown needles, mp 118—120°C.

Preparation Example 13

2,3,4-Trichloro-5-fluorobenzonitrile

To a suspension of 2,3,4-trichloro-5-fluoroaniline (43.8 g) in concentrated hydrochloric acid (300 ml) with vigorous stirring was added sodium nitrite (21.1 g) in water (50 ml) at −2 ~ 0°C for 20 minutes. After stirred for 30 minutes, the mixture was poured into ice water (300 ml) containing sodium tetrafluoroborate (67.2 g), stirred vigorously and then allowed to stand for 30 minutes in an ice bath. The resulting precipitate was collected by filtration and successively washed with chilled water and ether. This faint yellow precipitate was added portionwise during 30 minutes to a solution of cuprous cyanide (36.5 g), potassium cyanide (53.0 g) and sodium carbonate (11.1 g) in water (300 ml) with vigorous stirring at room temperature. After the mixture was stirred for 30 minutes, benzene (300 ml) was added to the suspension and then the mixture was stirred for 15 minutes. The insoluble material was collected by filtration, and washed with benzene (150 ml). The filtrate and washings wee combined and successively washed with 20% aqueous potassium cyanide and water, dried over anhydrous sodium sulfate and then concentrated. The resulting residue was recrystallized from n-hexane to give the title compound (27 g) as light brown needles, mp 97—99°C.

Preparation Example 14

3-Chloro-2,4,5-trifluorobenzonitrile

To a solution of potassium fluoride (31.7 g) in dimethyl sulfoxide (100 ml) with stirring at 130°C was added 2,3,4-trichloro-5-fluorobenzonitrile (15 g) and then the mixture was stirred for 1.5 hours at 140°C.

After cooling, the reaction mixture was poured into ice water (300 ml) and extracted with dichloromethane. The organic layer was washed with water, dried over anhydrous sodium sulfate and concentrated to give the title compound (11.9 g) as pale brown oil.

Preparation Example 15

3-Chloro-2,4,5-trifluorobenzamide

A solution of 3-chloro-2,4,5-trifluorobenzonitrile (11.9 g) in 30% hydrogen bromide-acetic acid (150 ml) was refluxed for 80 minutes, poured into ice water (350 ml) and extracted wth ether. The ether layer was successively washed with 1N-potassium hydroxide solution and water, dried over anhydrous sodium sulfate and concentrated. The residue was purified by silica gel chromatography eluting with n-hexane-ethyl acetate to give the title compound (3.97 g), mp 110—113.5°C.

Preparation Example 16

3-Chloro-2,4,5-trifluorobenzoic acid

A mixture of 3-chloro-2,4,5-trifluorobenzamide (3.97 g) and 18N-sulfuric acid (20 ml) was stirred at 125—135°C for 9 hours, and then poured into ice water (100 ml). After standing overnight, the resulting precipitate was collected by filtration. The mother liquor was extracted with ether, and the ether layer was dried over anhydrous sodium sulfate, then concentrated and mixed to the precipitate. A solution of the above precipitate and residue in dichloromethane (150 ml) was filtered through a celite pad and the filtrate was concentrated to give the title compound (2.83 g), mp 115—116.5°C.

Analysis (%) for $C_7H_2ClF_3O_2$, Calcd. (Found): C, 39.93 (40.18); H, 0.96 (0.80).

Preparation Example 17

3-Chloro-2,4,5-trifluorobenzoyl chloride

A solution of the 3-chloro-2,4,5-trifluorobenzoic acid (2.38 g) in thionyl chloride (10 ml) was refluxed for 2.5 hours, and then concentrated. The resulting residue was purified by distillation in nitrogen atmosphere to give the title compound (1.99 g), bp 88°C/25 mbar.

Preparation Example 18

Diethyl 3-chloro-2,4,5-trifluorobenzoylmalonate

Magnesium turnings (0.22 g and carbon tetrachloride (0.1 ml) was added to absolute ethanol (1.5 ml). To the stirring suspension was added dropwise a solution of diethyl malonate (1.4 g) and absolute ethanol (2 ml) in toluene (6 ml) during 28 minutes at 47—60°C. The mixture was stirred for 80 minutes, and then cooled in an acetone-dry ice bath. A solution of 3-chloro-2,4,5-trifluorobenzoyl chloride (1.99 g) in anhydrous toluene (2 ml) was added dropwise to the resulting solution at $-12 \sim -8$°C during 13 minutes. The mixture was stirred for 2 hours at $-10 \sim -5$°C allowed to stand overnight at room temperature, and then mixed with ice water (6 ml) containing concentrated sulfuric acid (0.4 ml). The resulting organic layer was collected and the water layer was extracted with toluene. The combined organic layer was washed with water, dried over anhydrous sodium sulfate and then concentrated to give the title compound (3.05 g) as pale yellow oil.

Preparation Example 19

Ethyl 3-chloro-2,4,5-trifluorobenzoylacetate

To an emulsion of diethyl 3-chloro-2,4,5-trifluorobenzoylmalonate (3.05 g) in water (4 ml) was added p-toluenesulfonic acid (4 mg) and refluxed for 4 hours with vigorous stirring. After cooling; the reaction mixture was extracted with dichloromethane. The organic layer was washed with water, dried over anhydrous sodium sulfate and concentrated. The residue was recrystallized from ether-n-hexane to give the title compound (1.22 g). mp 80—83°C.

Analysis (%) for $C_{11}H_8ClF_3O_3$, Calcd. (Found): C, 47.08 (46.96); H, 2.87 (2.77).

Preparation Example 20

Ethyl 2-(3-chloro-2,4,5-trifluorobenzoyl-3-ethoxyacrylate

A mixture of ethyl 3-chloro-2,4,5-trifluorobenzoylacetate (1.22 g), ethyl orthoformate (0.97 g) and acetic anhydride (1.12 g) was stirred at 118—143°C for 3 hours and then concentrated to give the title compound (1.4 g) as yellow oil.

Preparation Example 21

Ethyl 2-(3-chloro-2,4,5-trifluorobenzoyl)-3-cyclopropylaminoacrylate

To a solution of ethyl 2-(3-chloro-2,4,5-trifluorobenzoyl)-3-ethoxyacrylate (1.4 g) in absolute ethanol (3 ml) was added a solution of cyclopropylamine (0.26 g) in absolute ethanol (2 ml) at 5—10°C during 15 minutes. The mixture was allowed to stand at 5°C for 1.5 hours and then stirred at room temperature for an hour. The resulting precipitate was collected by filtration. The filtrate was concentrated, mixed with the precipitate and then recrystallized from petroleum ether to give the title compound (1.09 g), mp 84—85.5°C.

Analysis (%) for $C_{15}H_{13}ClF_3NO_3$, Calcd. (Found): C, 51.81 (51.76); H, 3.77 (3.74); N, 4.03 (4.03).

Preparation Example 22

Ethyl 8-chloro-1-cyclopropyl-6,7-difluoro-1,4-dihydro-4-oxo-3-quinolinecarboxylate

To a solution of ethyl 2-(3-chloro-2,4,5-trifluorobenzoyl)-3-cyclopropylaminoacrylate (1.09 g) in anhydrous dimethylformamide (5 ml) was added sodium fluoride (0.21 g). The mixture was stirred at 130—156°C for 3.5 hours, and then poured into ice water (50 ml) and the resulting precipitate was collected by filtration, washed with water and recrystallized from ethyl acetate to give the title compound (0.96 g), mp 158—159°C.

Analysis (%) for $C_{15}H_{12}ClF_2NO_3$, Calcd. (Found): C, 54.98 (54.96); H, 3.69 (3.57); N, 4.27 (4.25).

Preparation Example 23

8-Chloro-1-cyclopropyl-6,7-difluoro-1,4-dihydro-4-oxo-3-quinolinecarboxylic acid

A mixture of ethyl 8-chloro-1-cyclopropyl-6,7-difluoro-1,4-dihydro-4-oxo-3-quinolinecarboxylate (0.24 g), acetic acid (2 ml), water (1.5 ml) and concentrated sulfuric acid (0.25 ml) was refluxed for an hour, and then poured into ice water. The resulting precipitate was collected by filtration and successively washed with water and ether to give the title compound (0.17 g), mp 194—195°C.

Analysis (%) for $C_{13}H_8ClF_2NO_3$, Calcd. (Found): C, 52.11 (52.00); H, 2.69 (2.53); N, 4.67 (4.64).

Example 2

8-Chloro-1-cyclopropyl-6-fluoro-1,4-dihydro-7-(3-ethylaminomethyl-1-pyrrolidinyl)-4-oxo-3-quinolinecarboxylic acid

A mixture of 8-chloro-1-cyclopropyl-6,7-difluoro-1,4-dihydro-4-oxo-3-quinolinecarboxylic acid (0.4 g), 3-ethylaminomethylpyrrolidine (0.19 g) and DBU (0.2 g) in acetonitrile (4 ml) was heated to reflux for 2 hours and then allowed to stand overnight during which time a crystal separated. The crystal, title compound, was filtered and recrystallized from chloroform-methanol giving colorless prisms (0.39 g), mp 250—252°C (decompd.).

Analysis (%) for $C_{20}H_{23}ClFN_3O_3 \cdot 1/2\ H_2O$, Calcd. (Found): C, 57.62 (57.48); H, 5.80 (5.52); N, 10.08 (10.07).

Example 3

8-Chloro-1-cyclopropyl-6-fluoro-1,4-dihydro-7-(3-aminomethyl-1-pyrrolidinyl)-4-oxo-3-quinolinecarboxylic acid hydrochloride

A mixture of 8-chloro-1-cyclopropyl-6,7-difluoro-1,4-dihydro-4-oxo-3-quinolinecarboxylic acid (0.35 g), 3-aminomethylpyrrolidine (0.13 g), and DBU (0.19 g) in acetonitrile (4 ml) was refluxed for 2 hours and then allowed to stand overnight. A solid separated was collected by filtration and recrystallized from chloroform-methanol-n-hexane giving the crystalline product which was further purified by silica gel column chromatography using chloroform-methanol-concentrated aqueous ammonia (20:6:1) as eluent to obtain the free form (188 mg) of the title compound. The compound was dissolved in a mixture of methanol and concentrated to dryness. The residue was successively recrystallized from ethanol-acetonitrile and ethanol to give the title compound (14 mg) as pale yellow prisms, mp 238—247°C (decompd).

Analysis (%) for $C_{18}H_{19}ClFN_3O_2 \cdot HCl$, Calcd. (Found): C, 51.94 (51.63); H, 4.84 (5.01); N, 10.09 (9.85).

Example 4

7-(3-Aminomethyl-1-pyrrolidinyl)-8-bromo-1-cyclopropyl-6-fluoro-1,4-dihydro-4-oxo-3-quinolinecarboxylic acid

A mixture of 8-bromo-1-cyclopropyl-6,7-difluoro-1,4-dihydro-4-oxo-3-quinolinecarboxylic acid (200 mg), 3-aminomethylpyrrolidine (60 mg) and DBU (90 mg) in acetonitrile (3 ml) was stirred for an hour under refluxing and then for further 4 hours at room temperature. The crystals which separated were collected and recrystallized from dichloromethane to give the title compound (40 mg) as pale yellow prisms, mp 222—230°C (decompd.).

Analysis (%) for $C_{18}H_{19}BrFN_3O_3 \cdot H_2O$, Calcd. (Found): C, 48.80 (48.80); H, 4.79 (4.74); N, 9.50 (9.45).

In this example, the starting material is also novel and it is synthesized by following process.

Preparation Example 24

2-Bromo-3-chloro-4-fluoro-6-nitroaniline

Into a solution of 3-chloro-4-fluoro-6-nitroaniline (200.3 g) in acetic acid (1.5 liter) was added bromine (339 g) during a period of 80 minutes at 50°C under stirring and stirred for further 2 hours. The reaction mixture was poured into ice water (3 liter) and the resulting precipitate was collected by filtration, washed with water and added to a mixture of concentrated hydrochloric acid (300 ml) and ethanol (1.2 liter). The mixture was refluxed for 8.5 hrs. After cooling, the precipitate was collected by filtration and washed with water and dried. The title compound thus obtained weighed 235.6 g as yellow needles, mp 146—147°C.

Preparation Example 25

3-Bromo-2,4-dichloro-5-fluoronitrobenzene

To a mixture of anhydrous cupric chloride (147 g) and 2-bromo-3-chloro-4-fluoro-6-nitroaniline (235.6 g) in anhydrous acetonitrile (1.5 liter) was added *tert*-butylnitrite (135.2 g) at 60°C during 70 minutes. The

reaction mixture was poured into ice-chilled diluted hydrochloric acid (1.5 liter) and extracted with benzene. The organic layer was successively washed with ice-chilled diluted hydrochloric acid and water saturated with sodium chloride, dried over anhydrous sodium sulfate and concentrated. The resulting residue was purified by distillation to give the title compound (218.8 g), bp 78—117°C/2,66 mbar. The oil was crystallized from methanol to give yellow prisms, mp 65.5—67.5°C.

Preparation Example 26

3-Bromo-2,4-dichloro-5-fluoroaniline

To a suspension of iron powder (135.4 g) in water (140 ml), with vigorous stirring at 50—60°C, was slowly added concentrated hydrochloric acid (18 ml). After ethanol (350 ml) was mixed, 3-bromo-2,4-dichloro-5-fluoronitrobenzene (218.8 g) was added portionwise to the suspension at 52—76°C during an hour. After stirring for 75 minutes at the same temperature, the hot reaction mixture was filtered after adding benzene (500 ml) and the insoluble material was successively washed with hot ethanol (100 ml) and benzene (200 ml). The filtrate and washings were combined. The organic layers were washed with water saturated with sodium chloride, dried over anhydrous sodium sulfate and then concentrated. The resulting residue was recrystallized from ethanol-water to give the title compound (141.6 g) as light brown needles, mp 126—129.5°C.

Preparation Example 27

3-Bromo-2,4-dichloro-5-fluorobenzonitrile

To a suspension of 3-bromo-2,4-dichloro-5-fluoroaniline (141.6 g) in concentrated hydrochloric acid (900 ml) with vigorous stirring was added sodium nitrite (56.6 g) in water (120 ml) at −2 ~ 0°C for 40 minutes. After stirring for 30 minutes, the mixture was poured into ice water (700 ml) containing sodium tetrafluoroborate (180 g), stirred vigorously for 20 minutes and then allowed to stand for 15 minutes in an ice bath. The resulting precipitate was collected by filtration and washed with chilled water. The wet crude tetrafluoroborate thus obtained weighed 270.8 g. The borate was added portionwise during 45 minutes to a solution of cuprous cyanide (98 g), potassium cyanide (142.4 g) and sodium carbonate (29 g) in water (800 ml) with vigorous stirring at 9—10°C. After the mixture was stirred for 2 hours at room temperature, benzene (700 ml) and potassium cyanide (71 g) were added to the suspension and then the mixture was stirred for 30 minutes. The insoluble material was collected by filtration, and washed with benzene (300 ml × 2). The filtrate and washings were combined and washed five times with water saturated with sodium chloride, dried over anhydrous sodium sulfate and then concentrated. The resulting residue was recrystallized from ethanol to give the title compund (75.5 g) as red brown prisms, mp 110.6—112.5°C.

Preparation Example 28

3-Bromo-2,4,5-trifluorobenzonitrile

To a solution of potassium fluoride (123 g) in dimethyl sulfoxide (400 ml) with stirring at 133°C was added 3-bromo-2,4-dichloro-5-fluorobenzonitrile (68.4 g) and then the mixture was stirred for 5 hours and 20 minutes at 130°C. After cooling, the reaction mixture was poured into ice water (1 liter) and extracted with benzene. The organic layer was washed wth water saturated with sodium chloride, dried over anhydrous sodium sulfate and distilled to give the title compound (15.7 g) as colorless oil, bp 82.5°C/17 mbar—80.0°C/1,33 mbar.

Preparation Example 29

3-Bromo-2,4,5-trifluorobenzoic acid

A mixture of 3-bromo-2,4,5-trifluorobenzonitrile (13.9 g) in concentrated sulfuric acid (8 ml) was heated for 20 minutes on an oil bath (100°C), poured into ice water (350 ml). The resulting precipitate was collected by filtration and washed with water. The filtrate and washings were extracted 3 times with dichloromethane. The dichloromethane layer was washed with water saturated with sodium chloride, dried over anhydrous sodium sulfate and concentrated to give the residue. The combined mixture of the precipitate obtained previously and the residue was purified by silica gel chromatography eluting with dichloromethane → dichloromethane:methanol (10:1) to give 3-bromo-2,4,5-trifluorobenzamide (8.7 g).

A mixture of 3-bromo-2,4,5-trifluorobenzamide (8.7 g) and 18N-sulfuric acid (50 ml) was stirred at 100°C for 4 hours, and then poured into ice water (200 ml). The resulting precipitate was collected by filtration and recrystallized from dichloromethane-n-hexane to give the title compound (6.9 g), mp 125—127°C.

Preparation Example 30

3-Bromo-2,4,5-trifluorobenzoyl chloride

A solution of the 3-bromo-2,4,5-trifluorobenzoic acid (2.5 g) in thionyl chloride (10 ml) was refluxed for 2.5 hours, and then concentrated. The resulting residue was purififed by distillation through Widmer fractionating column to give the title compound (2.3 g), bp 98—102°C/24 mbar.

Preparation Example 31

Diethyl 3-bromo-2,4,5-trifluorobenzoylmalonate

Magnesium turnings (0.22 g) and carbon tetrachloride (0.1 ml) was added to absolute ethanol (1.5 ml).

To the stirring suspension was added dropwise a solution of diethyl malonate (1.4 g) and absolute ethanol (2 ml) in toluene (6 ml) during 25 minutes at 50—60°C. The mixture was stirred for 40 minutes, and then cooled. A solution of 3-bromo-2,4,5-trifluorobenzoyl chloride (2.27 g) in anhydrous toluene (3 ml) was added dropwise to the solution at −8 ~ −4.5°C during 28 minutes. The mixture was stirred for 2 hours and then mixed with ice-chilled diluted sulfuric acid. The resulting organic layer was collected and the water layer was extracted with toluene (6 ml × 4). The combined organic layer was washed with water, dried over anhydrous sodium sulfate and then concentrated to give the title compound (3.25 g) as pale yellow oil.

Preparation Example 32

Ethyl 3-bromo-2,4,5-trifluorobenzoylacetate

To an emulsion of diethyl 3-bromo-2,4,5-trifluorobenzoylmalonate (3.25 g) in water (4 ml) was added p-toluenesulfonic acid (4 mg) and refluxed for 3 hours with vigorous stirring. After cooling, the reaction mixture was extracted with dichloromethane (8 ml × 4). The organic layer was washed with water saturated with sodium chloride, dried over anhydrous sodium sulfate and concentrated. The residue was recrystallized from dichloromethane-n-hexane to give the title compound (1.51 g), mp 85—88°C.

Preparation Example 33

Ethyl 2-(3-bromo-2,4,5-trifluorobenzoyl)-3-ethoxyacrylate

A mixture of ethyl 3-bromo-2,4,5-trifluorobenzoylacetate (1.5 g), ethyl orthoformate (1.0 g) and acetic anhydride (1.2 g) was stirred at 130°C for 4.5 hours and then concentrated to give the title compound (1.75 g) as yellow oil.

Preparation Example 34

Ethyl 2-(3-bromo-2,4,5-trifluorobenzoyl)-3-cyclopropylaminoacrylate

To a solution of ethyl 2-(3-bromo-2,4,5-trifluorobenzoyl)-3-ethoxyacrylate (1.75 g) in absolute ethanol (5 ml) was added a solution of cyclopropylamine (0.32 g) in absolute ethanol (2 ml) under ice-cooling during 30 minutes. The mixture was stirred at 5—20°C for 2.5 hours and concentrated. The residue was recrystallized from petroleum ether to give the title compound (1.36 g), mp 74—76°C.

Preparation Example 35

Ethyl 8-bromo-1-cyclopropyl-6,7-difluoro-1,4-dihydro-4-oxo-3-quinolinecarboxylate

To a solution of ethyl 2-(3-bromo-2,4,5-trifluorobenzoyl)-3-cyclopropylaminoacrylate (1.35 g) in anhydrous dimethylformamide (5 ml) was added sodium fluoride (0.23 g). The mixture was stirred at 97—108°C for 7.5 hours, and then poured into ice water (50 ml) and the resulting precipitate was collected by filtration, washed with water and recrystallized from dichloromethane-n-hexane to give the title compound (1.05 g), mp 163.5—168°C as colorless prisms.

Preparation Example 36

8-Bromo-1-cyclopropyl-6,7-difluoro-1,4-dihydro-4-oxo-3-quinolinecarboxylic acid

A mixture of ethyl 8-bromo-1-cyclopropyl-6,7-difluoro-1,4-dihydro-4-oxo-3-quinolinecarboxylate (1.0 g), acetic acid (4 ml), water (3 ml) and concentrated sulfuric acid (0.5 ml) was heated on an oil bath (90—100°C) for an hour under stirring, then for an hour at room temperature and poured into ice water (20 ml). The resulting precipitate was collected by filtration and washed with water to give the title compound (0.82 g), mp 224—225.5°C.

Example 5

8-Bromo-1-cyclopropyl-6-fluoro-1,4-dihydro-7-(3-methylaminomethyl-1-pyrrolidinyl)-4-oxo-3-quinolinecarboxylic acid

A mixture of 8-bromo-1-cyclopropyl-6,7-difluoro-1,4-dihydro-4-oxo-3-quinolinecarboxylic acid (200 mg), 3-methylaminomethylpyrrolidine (90 mg) and DBU (100 mg) in acetonitrile (3 ml) was stirred for an hour under refluxing and then for further 3 hours at room temperature. The crystals which separated were collected and recrystallized from chloroform-methanol-ammonia to give the title compound (160 mg) as pale yellow prisms, mp 242.5—246°C (decompd.).

Analysis (%) for $C_{19}H_{21}BrFN_3O_3$, Calcd. (Found): C, 52.07 (52.28); H, 4.83 (4.85); N, 9.59 (9.61).

Example 6

8-Bromo-1-cyclopropyl-7-(3-ethylaminomethyl-1-pyrrolidinyl)-6-fluoro-1,4-dihydro-4-oxo-3-quinolinecarboxylic acid

A mixture of 8-bromo-1-cyclopropyl-6,7-difluoro-1,4-dihydro-4-oxo-3-quinolinecarboxylic acid (280 mg), 3-ethylaminomethylpyrrolidine (90 mg) and DBU (90 mg) in acetonitrile (3 ml) was stirred for an hour under refluxing and then for further 3 hours at room temperature. The crystals which separated were collected and recrystallized from chloroform-methanol-ammonia to give the title compound (150 mg) as colorless prisms, mp 258—260°C (decompd.).

Analysis (%) for $C_{20}H_{23}BrFN_3O_3$, Calcd. (Found): C, 53.11 (53.54); H, 5.12 (5.11); N, 9.29 (9.43).

Example 7

Ethyl 8-chloro-1-cyclopropyl-7-(3-ethylaminomethyl-1-pyrrolidinyl)-6-fluoro-1,4-dihydro-4-oxo-3-quinolinecarboxylate

A mixture of ethyl 8-chloro-1-cyclopropyl-6,7-difluoro-1,4-dihydro-4-oxo-3-quinolinecarboxylate (500 mg), acetonitrile (5 ml), 3-ethylaminomethylpyrrolidine (296 mg) and DBU (233 mg) was refluxed for 5 hours and then concentrated to give the residue, to which ice water (20 ml) was added. The mixture was extracted with chloroform. The extract was washed with water, dried over anhydrous sodium sulfate and concentrated to give the title compound (800 mg) as brown oil.

IR ($cm^{-1}$): 3010, 1720, 1610, 1450, 1315.

NMR (δ in $CDCl_3$):

0.9—1.3 (4H, m, 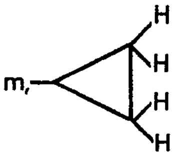 ), 1.12 (3H, t, —N—$CH_2CH_3$), 1.39 (3H, t, —O—$CH_2CH_3$),

1.5—1.9 (1H, m 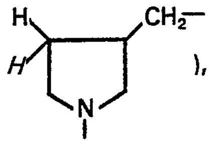 ), 2.0—2.2 (1H, m, 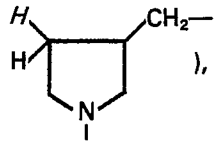 ),

2.2—2.55 (1H, m 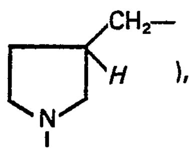 ), 2.68 (4H, m, 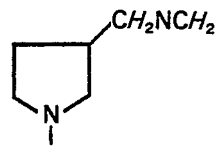 ),

3.3—3.7 (4H, m, 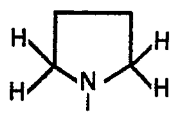 ), 4.17 (1H, m, 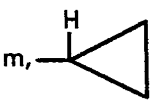 ),

4.37 (2H, q, —$CH_2CH_3$), 7.93 (1H, d, J=13.6Hz, 5—H), 8.60 (1H, s, 2—H).

Example 8

8-Chloro-1-cyclopropyl-7-(3-ethylaminomethyl-1-pyrrolidinyl)-6-fluoro-1,4-dihydro-4-oxo-3-quinolinecarboxylic acid

A mixture of ethyl 8-chloro-1-cyclopropyl-7-(3-ethylaminomethyl-1-pyrrolidinyl)-6-fluoro-1,4-dihydro-4-oxo-3-quinolinecarboxylate (800 mg) and 1N sodium hydroxide (8 ml) was refluxed for 1⅓ hours and concentrated. The residue was purified by silica gel column chromatography ($CHCl_3$:MeOH = 4:1) to give yellow crystalline powder. The powder was suspended in acetonitrile, then collected by filtration and recrystallized from chloroform-methanol to give the title compound (130 mg) as yellow prisms, mp 248—251°C (decompd.).

Analysis (%) for $C_{20}H_{23}ClFN_3O_3 \cdot 1/4\ H_2O$, Calcd. (Found): C, 58.25 (58.46); H, 5.74 (5.65); N, 10.19 (10.26).

Example 9

8-Chloro-1-cyclopropyl-6-fluoro-1,4-dihydro-7-(3-dimethylaminomethyl-1-pyrrolidinyl)-4-oxo-3-quinolinecarboxylic acid

A mixture of 8-chloro-1-cyclopropyl-6,7-difluoro-1,4-dihydro-4-oxo-3-quinolinecarboxylic acid (500 mg), 3-dimethylaminomethylpyrrolidine (330 mg), DBU (250 mg) and acetonitrile (5 ml) was refluxed for an hour under stirring, then allowed to stand for overnight and concentrated. To the resulting residue was added methanol and the mixture was filtered to collect the crystalline product which was recrystallized from chloroform-methanol to give the title compound (430 mg) as yellow prisms, mp 175—176°C.

Analysis (%) for $C_{20}H_{23}ClFN_3O_3$, Calcd. (Found): C, 58.90 (59.02); H, 5.68 (5.70); N, 10.30 (10.19).

Reference Example 1

7-(3-Aminomethyl-1-pyrrolidinyl)-1-cyclopropyl-6,8-difluoro-1,4-dihydro-4-oxo-3-quinolinecarboxylic acid hydrochloride

A mixture of ethyl 1-cyclopropyl-6,7,8-trifluoro-1,4-dihydro-4-oxo-3-quinolinecarboxylate (0.3 g), 1,8-diazabicyclo-(5,4,0)-7-undecene (DBU, 0.15 g) and 3-aminomethylpyrrolidine (0.14 g) in acetonitrile (5 ml) was refluxed under stirring for 3.5 hours. After cooling, the mixture was poured into ice-water (50 ml) and

extracted three times with chloroform (50 ml). The extracts were washed with water, dried over anhydrous sodium sulfate and concentrated to give the residue, ethyl 7-(3-aminomethyl-1-pyrrolidinyl)-1-cyclopropyl-6,8-difluoro-4-oxo-3-quinolinecarboxylate.

To the residue was added 1N-sodium hydroxide solution (6 ml) and the mixture was stirred at 100°C for an hour. After cooling, the mixture was neutralized by adding acetic acid and concentrated. Purification of the residue by silica gel column chromatography ($CHCl_3$:MeOH:con.$NH_4OH$ = 10:3:3) gave the title compound (54 mg) as pale yellow crystals, mp 248—250°C, after recrystallization from acetone-HCl (1%).

Analysis (%) for $C_{18}H_{19}F_2N_3O_3 \cdot HCl \cdot 1/3\ H_2O$; Calcd. (Found): C, 53.27 (53.40); H, 5.13 (4.98); N, 10.35 (10.35).

Reference Example 2

1-Cyclopropyl-7-(3-ethylaminomethyl-1-pyrrolidinyl)-6,8-difluoro-1,4-dihydro-4-oxo-3-quinolinecarboxylic acid hydrochloride

A mixture of ethyl 1-cyclopropyl-6,7,8-trifluoro-1,4-dihydro-4-oxo-3-quinolinecarboxylate (0.3 g), DBU (0.15 g) and 3-ethylaminomethylpyrrolidine (0.13 g) in acetonitrile (5 ml) was refluxed under stirring for 3.5 hours. After cooling, the reaction mixture was poured into ice-water (25 ml) and extracted with chloroform (50 ml). The extract was washed with water, dried over anhydrous sodium sulfate and concentrated. To the residue, ethyl cyclopropyl - 7 - (3 - ethylaminomethyl - 1 - pyrrolidinyl) - 6,8 - difluoro - 4 - oxo - 3 - quinolinecarboxylate, was added 1N-sodium hydroxide solution (6 ml) and the mixture was stirred at 70 to 80°C for an hour. After cooling, the mixture was neutralized by adding acetic acid and concentrated. The residue, after purified by silica gel column chromatography ($CHCl_3$:MeOH:con.$NH_4OH$=10:10:3), was dissolved in methanol containing hydrochloric acid. The solution was concentrated to dryness and the solid was recrystallized from methanol to give the title compound (0.11 g), mp 258—260°C.

Analysis (%) for $C_{20}H_{23}F_2N_3O_3 \cdot HCl$; Calcd. (Found): C, 56.14 (55.75); H, 5.65 (5.57); N, 9.82 (9.81).

Reference Example 3

1-Cyclopropyl-6,7,8-trifluoro-1,4-dihydro-4-oxo-3-quinolinecarboxylic acid

A mixture of ethyl 1-cyclopropyl-6,7,8-trifluoro-1,4-dihydro-4-oxo-3-quinolinecarboxylate (3.0 g), acetic acid (20 ml), sulfuric acid (2.5 ml) and water (15 ml) was refluxed under stirring for an hour. After cooling the reaction mixture was poured into ice-water. The precipitate was filtered, washed sufficiently with water and dried in vacuum to give the title compound (2.59 g) as colorless needles, mp 231—232°C.

Analysis (%) for $C_{13}H_8F_3NO_3$; Calcd. (Found): C, 55.13 (55.11); H, 2.85 (2.61); N, 4.95 (4.79).

Reference Example 4

7-(3-Aminomethyl-1-pyrrolidinyl)-1-cyclopropyl-6,8-difluoro-1,4-dihydro-4-oxo-3-quinolinecarboxylic acid

A mixture of 1-cyclopropyl-6,7,8-trifluoro-1,4-dihydro-4-oxo-3-quinolinecarboxylic acid (1.2 g), DBU (0.15 g) and 3-aminomethylpyrrolidine (0.45 g) in anhydrous acetonitrile (10 ml) was stirred under reflux for 1.5 hours and then at room temperature for 9.5 hours. The precipitate formed was filtered and recrystallized from dichloromethane-methanol (1:1). The title compound (0.88 g), mp 235—236°C, was obtained as colorless prisms.

Analysis (%) for $C_{18}H_{19}F_2N_3O_3$; Calcd. (Found): C, 59.50 (59.45); H, 5.27 (5.17); N, 11.56 (11.53).

Reference Example 5

1-Cyclopropyl-7-(3-ethylaminomethyl-1-pyrrolidinyl)-6,8-difluoro-1,4-dihydro-4-oxo-3-quinolinecarboxylic acid hydrochloride

A mixture of 1-cyclopropyl-6,7,8-trifluoro-1,4-dihydro-4-oxo-3-quinolinecarboxylic acid (1.2 g), DBU (0.65 g) and 3-ethylaminomethylpyrrolidine (0.57 g) in anhydrous acetonitrile (10 ml) was stirred under reflux for 1.5 hours and then at room temperature for 9.5 hours. The precipitate formed was filtered and dissolved in methanol containing hydrochloric acid. The solution was concentrated and the residue was recrystallized from water-ethanol to give the title compound (0.7 g) as pale yellow prisms, mp 256—258.5°C.

Analysis (%) for $C_{20}H_{23}F_2N_3O_3 \cdot HCl$; Calcd. (Found): C, 56.14 (56.04); H, 5.65 (5.64); N, 9.82 (9.74).

Reference Example 6

7-(3-Aminomethyl-1-pyrrolidinyl)-1-cyclopropyl-6-fluoro-1,4-dihydro-4-oxo-3-quinolinecarboxylic acid

A mixture of 7-chloro-1-cyclopropyl-6-fluoro-1,4-dihydro-4-oxo-3-quinolinecarboxylic acid (0.5 g) and 3-aminomethylpyrrolidine (0.55 g) in β-picoline (18 ml) was refluxed for 3 hours under stirring. After cooling, to the mixture was added concentrated aqueous ammonia. The resulting mixture was concentrated to give the residue, to which acetonitrile (50 ml) was added. The precipitate was filtered, washed successively with ethanol-ether (1:1) and ether and recrystallized from methanol to give the title compound (0.12 g) as pale yellow prisms, mp 241—246°C.

Analysis (%) for $C_{18}H_{20}FN_3O_3$; Calcd. (Found): C, 62.60 (62.30); H, 5.84 (5.71); N, 12.17 (12.11).

Reference Example 7

1-Cyclopropyl-7-(3-ethylaminomethyl-1-pyrrolidinyl)-6-fluoro-1,4-dihydro-4-oxo-3-quinolinecarboxylic acid hydrochloride

A mixture of 7-chloro-1-cyclopropyl-6-fluoro-1,4-dihydro-4-oxo-3-quinolinecarboxylic acid (0.6 g) and 3-ethylaminomethylpyrrolidine (0.81 g) in β-picoline (15 ml) was refluxed for 2 hours under stirring. After cooling, concentrated aqueous ammonia (20 ml) was added to the reaction mixture. The mixture was concentrated and the residue was dissolved in a mixture of concentrated hydrochloric acid and methanol. Removal of the solvent left a solid which was recrystallized from water-methanol-ethyl acetate (1:1:1) to give the title compound (125 mg) as colorless prisms, mp 282—285°C (decompd.).

Analysis (%) for $C_{20}H_{24}FN_3O_3 \cdot HCl$; Calcd. (Found): C, 58.10 (58.18); H, 6.19 (6.38); N, 10.16 (10.20).

Reference Example 8

1-Cyclopropyl-6,8-difluoro-1,4-dihydro-7-(3-methylaminomethyl-1-pyrrolidinyl)-4-oxo-3-quinolinecarboxylic acid hydrochloride

A mixture of 1-cyclopropyl-6,7,8-trifluoro-1,4-dihydro-4-oxo-3-quinolinecarboxylic acid (0.6 g), 3-methylaminomethylpyrrolidine (0.28 g) and DBU (0.33 g) in acetonitrile (5 ml) was stirred under reflux for an hour and then at room temperature for 8 hours. The reaction mixture was treated by the same way as described in Example 5. The title compound was obtained as pale yellow prisms (0.46 g), mp 269—273°C (decompd.) after recrystallization from methanol.

Analysis (%) for $C_{19}H_{21}F_2N_3O_3 \cdot HCl$, Calcd. (Found): C, 55.14 (54.91); H, 5.36 (5.31); N, 10.15 (10.10).

Reference Example 9

1-Cyclopropyl-6-fluoro-1,4-dihydro-7-(3-methylaminomethyl-1-pyrroldinyl)-4-oxo-3-quinolinecarboxylic acid hydrochloride

A mixture of 7-chloro-1-cyclopropyl-6-fluoro-1,4-dihydro-4-oxo-3-quinolinecarboxylic acid (0.6 g) and 3-methylaminomethylpyrrolidine (0.75 g) in β-picoline (4 ml) was stirred under reflux for 50 minutes and then at room temperature for 3 hours. The reaction mixture was treated by the same way as shown in Example 7. The title compound was obtained as pale yellow prisms (290 mg), mp 265°C (decompd.) after recrystallization from water.

Analysis (%) for $C_{19}H_{22}FN_3O_3 \cdot HCl \cdot 1/5\ H_2O$, Calcd. (Found): C, 57.13 (57.04); H, 5.90 (5.83); N, 10.52 (10.36).

Reference Example 10

1-Cyclopropyl-6,8-difluoro-1,4-dihydro-4-oxo-7-(3-n-propylaminomethyl-1-pyrrolidinyl)-3-quinolinecarboxylic acid hydrochloride

A solution of ethyl 1-cyclopropyl-6,7,8-trifluoro-1,4-dihydro-4-oxo-3-quinolinecarboxylate (0.3 g) and 3-n-propylaminomethylpyrrolidine (0.55 g) in DMF (10 ml) was stirred at 80—90°C for 2 hours. After cooling, the mixture was poured into ice-water, alkalized by adding aqueous potassium carbonate solution and extracted with chloroform. The chloroform extract was dried over anhydrous sodium sulfate and then concentrated. Then residue, ethyl 1-cyclopropyl-6,8-difluoro-1,4-dihydro-4-oxo-7-(3-n-propylaminomethyl-1-pyrrolidinyl)-3-quinolinecarboxylate in aqueous 1N sodium hydroxide solution (10 ml) was heated to reflux for an hour and then neutralized by adding acetic acid to give a crystalline precipitate which was collected by filtration and recrystallized from ethanol-water-acetonitrile. The product was dissolved in methanol containing hydrochloric acid and the solution was concentrated. The resulting residue was recrystallized from methanol to give the title compound (47 mg) as fine brown crystals, mp 270—278°C (decompd.).

Analysis (%) for $C_{21}H_{25}F_2N_3O_3 \cdot HCl \cdot 4/5\ H_2O$, Calcd. (Found): C, 55.28 (55.13); H, 6.10 (5.76); N, 9.21 (9.13).

Reference Example 11

1-Cyclopropyl-6,8-difluoro-1,4-dihydro-7-(3-isopropylaminomethyl-1-pyrrolidinyl)-4-oxo-3-quinolinecarboxylic acid hydrochloride

A mixture of ethyl 1-cyclopropyl-6,7,8-trifluoro-1,4-dihydro-4-oxo-3-quinolinecarboxylate (0.15 g) and 3-isopropylaminomethylpyrrolidine (0.27 g) in DMF (10 ml) was stirred at 80—90°C for 2 hours. After cooling, the mixture was diluted with ice-water, alkalized by adding aqueous potassium carbonate solution and extracted with chloroform. The chloroform extract was dried over anhydrous sodium sulfate and then concentrated. The resulting residue, ethyl 1 - cyclopropyl - 6,8 - difluoro - 1,4 - dihydro - 7 - (3 - isopropylaminomethyl - 1 - pyrrolidinyl) - 4 - oxo - 3 - quinolinecarboxylate in aqueous 1N-sodium hydroxide solution was heated to reflux for an hour. The mixture was neutralized by adding acetic acid and concentrated. The residue was purified by silica gel column chromatography ($CHCl_3$:MeOH:con.$NH_4OH$=10:10:3). The product was dissolved in methanol containing hydrochloric acid. Removal of the solvent left the title compound which was recrystallized from methanol to give fine brown crystals (15 mg), mp 265—271°C (decompd.).

Analysis (%) for $C_{21}H_{25}F_2N_3O_3 \cdot HCl \cdot 1/2\ H_2O$, Calcd. (Found): C, 55.94 (56.04); H, 6.04 (5.85); N, 9.32 (9.31).

Reference Example 12

1-Cyclopropyl-6,8-difluoro-1,4-dihydro-7-(3-dimethylaminomethyl-1-pyrrolidinyl)-4-oxo-3-quinolinecarboxylic acid

A mixture of 1-cyclopropyl-6,7,8-trifluoro-1,4-dihydro-4-oxo-3-quinolinecarboxylic acid (500 mg), 3-dimethylaminomethylpyrrolidine (340 mg), DBU (270 mg) and acetonitrile (5 ml) was refluxed for an hour under stirring, allowed to stand for overnight and filtered to collect the crystalline product which was washed with acetonitrile and ether successively to give the title compound (420 mg) as pale yellow needles, mp 171—181°C.

Analysis (%) for $C_{20}H_{23}F_2N_3O_3$, Calcd. (Found): C, 61.37 (61.03); H, 5.92 (5.95); N, 10.74 (10.76).

Experiment 1

In vitro antibacterial activity

The in vitro antibacterial activity against gram-positive and gram-negative bacteria has been investigated.

The minimum inhibitory concentration (MIC) was determined in accordance with the method recommended by Japan Society of Chemotherapy.

The results of the tests are shown in Table 1 and 2.

In these experiments, the inventive compounds described in Examples 1 to 9 have antibacterial properties which are even more marked than those of their 8-hydrogen or 8-fluoro homologues, as shown in Table 1 and 2, against aerobic gram-positive bacteria and anaerobic bacteria.

TABLE 1-1

In vitro antibacterial activity (standard strain)

| Organism ($10^6$ cells/ml) | Gram | MIC (μg/ml) | | | | |
|---|---|---|---|---|---|---|
| | | Ref. 1 | Ref. 2 | Ref. 4 | Ref. 6 | Ref. 7 |
| Bacillus subtilis PCI 219 | + | 0.025 | 0.0125 | 0.025 | 0.05 | 0.025 |
| Staphylococcus aureus 209 P | + | 0.05 | 0.025 | 0.05 | 0.05 | 0.10 |
| S. aureus Smith | + | 0.05 | 0.025 | 0.05 | 0.20 | 0.10 |
| S. aureus IID 670 (Terajima) | + | 0.05 | 0.025 | 0.025 | 0.05 | 0.10 |
| S. epidermidis IID 866 | + | 0.05 | 0.05 | 0.025 | 0.05 | 0.05 |
| Streptococcus pyogenes S-8 | + | 0.05 | 0.10 | 0.05 | 0.10 | 0.39 |
| S. pyogenes IID 692 | + | 0.05 | 0.20 | 0.05 | 0.20 | 0.39 |
| S. pneumoniae IID 552 | + | 0.05 | 0.10 | 0.05 | 0.10 | 0.39 |
| S. faecalis IID 682 | + | 0.05 | 0.20 | 0.05 | 0.39 | 0.39 |
| Escherichia coli NIHJ JC-2 | - | 0.025 | 0.025 | 0.0125 | 0.05 | 0.05 |
| E. coli ATCC 10536 | - | 0.025 | 0.025 | 0.025 | 0.05 | 0.05 |
| E. coli ML 4707 | - | 0.025 | 0.025 | 0.025 | 0.05 | 0.05 |
| Proteus vulgaris IFO 3167 | - | 0.025 | 0.025 | 0.025 | 0.05 | 0.05 |
| P. mirabilis IID 994 | - | 0.05 | 0.05 | 0.05 | 0.10 | 0.20 |
| Morganella morganii IID 602 | - | 0.10 | 0.10 | 0.10 | 0.39 | 0.39 |
| Enterobacter cloacae IID 977 | - | 0.10 | 0.10 | 0.05 | 0.20 | 0.39 |
| Citrobacter freundii IID 976 | - | 0.05 | 0.05 | 0.05 | 0.10 | 0.10 |
| Klebsiella pneumoniae KY(GN)6445 | - | 0.05 | 0.05 | 0.05 | 0.10 | 0.10 |
| K. pneumoniae 1-220S | - | 0.05 | 0.10 | 0.05 | 0.20 | 0.20 |
| Salmonella entritidis IID 604 | - | 0.05 | 0.05 | 0.05 | 0.20 | 0.20 |
| Shigella sonnei IID 969 | - | 0.025 | 0.025 | 0.025 | 0.05 | 0.05 |
| Yersinia enterocolitica IID 981 | - | 0.05 | 0.10 | 0.05 | 0.20 | 0.39 |
| Serratia marcescens IID 618 | - | 0.20 | 0.10 | 0.10 | 0.39 | 0.39 |
| S. marcescens GN 7577 | - | 0.78 | 1.56 | 0.78 | 3.13 | 6.25 |
| Pseudomonas aeruginosa V-1 | - | 0.39 | 0.78 | - | 0.78 | 1.56 |
| P. aeruginosa IFO 12689 | - | 0.39 | 1.56 | 0.39 | 0.78 | 3.13 |
| P. aeruginosa IID 1210 | - | 0.78 | 3.13 | 0.78 | 3.13 | 3.13 |
| Acinetobacter anitratus IID 876 | - | 0.10 | 0.05 | 0.05 | 0.78 | 0.78 |
| Alcaligenes faecalis 0104002 | - | 0.39 | 0.10 | 0.78 | 1.56 | 1.56 |
| Bacteroides fragilis GM 7000 | - | 0.78 | 1.56 | - | 6.25 | 25 |
| B. fragilis 0558 | - | 0.39 | 0.78 | 0.78 | 6.25 | 25 |
| B. fragilis 25285 | - | 0.39 | 0.78 | 0.78 | 12.5 | 25 |
| Fusobacterium varium KYA 8501 | - | 0.78 | 1.56 | 1.56 | 25 | 12.5 |
| Clostridium perfringens KYA 13123 | + | 0.39 | 0.39 | - | 3.13 | 0.20 |
| C. ramosum | + | 0.78 | 0.78 | 0.78 | 12.5 | 6.25 |
| C. difficile I-E | + | 1.56 | 0.78 | - | - | 12.5 |

TABLE 1-2

In vitro antibacterial activity (standard strain)

| Organism ($10^6$ cells/ml) | Gram | MIC (µg/ml) | | | | |
|---|---|---|---|---|---|---|
| | | Ref. 8 | Ref. 9 | Ref. 10 | Ref. 11 | Exp. 1 |
| Bacillus subtilis PCI 219 | + | 0.0125 | 0.05 | 0.0125 | 0.025 | 0.0125 |
| Staphylococcus aureus 209 P | + | 0.025 | 0.20 | 0.05 | 0.05 | 0.0125 |
| S. aureus Smith | + | 0.025 | 0.39 | 0.05 | 0.05 | 0.025 |
| S. aureus IID 670 (Terajima) | + | 0.025 | 0.20 | 0.05 | 0.05 | 0.025 |
| S. epidermidis IID 866 | + | 0.025 | 0.10 | 0.10 | 0.05 | 0.025 |
| Streptococcus pyogenes S-8 | + | 0.05 | 0.20 | 0.39 | 0.20 | 0.05 |
| S. pyogenes IID 692 | + | 0.05 | 0.20 | 0.39 | 0.20 | 0.05 |
| S. pneumoniae IID 552 | + | 0.05 | 0.39 | 0.39 | 0.39 | 0.05 |
| S. faecalis IID 682 | + | 0.10 | 0.39 | 0.39 | 0.39 | 0.05 |
| Escherichia coli NIHJ JC-2 | - | 0.025 | 0.05 | 0.05 | 0.05 | 0.025 |
| E. coli ATCC 10536 | - | 0.025 | 0.10 | 0.10 | 0.05 | 0.025 |
| E. coli ML 4707 | - | 0.025 | 0.10 | 0.20 | 0.05 | 0.025 |
| Proteus vulgaris IFO 3167 | - | 0.025 | 0.10 | 0.025 | 0.05 | 0.025 |
| P. mirabilis IID 994 | - | 0.05 | 0.39 | 0.025 | 0.10 | 0.025 |
| Morganella morganii IID 602 | - | 0.20 | 0.78 | 0.025 | 0.39 | 0.10 |
| Enterobacter cloacae IID 977 | - | 0.10 | 0.39 | 0.20 | 0.39 | 0.05 |
| Citrobacter freundii IID 976 | - | 0.025 | 0.20 | 0.05 | 0.10 | 0.05 |
| Klebsiella pneumoniae KY(GN)6445 | - | 0.025 | 0.10 | 0.05 | 0.10 | 0.05 |
| K. pneumoniae 1-220S | - | 0.10 | 0.39 | 0.20 | 0.20 | 0.05 |
| Salmonella entritidis IID 604 | - | 0.05 | 0.20 | 0.10 | 0.20 | 0.05 |
| Shigella sonnei IID 969 | - | 0.0125 | 0.10 | 0.025 | 0.05 | 0.025 |
| Yersinia enterocolitica IID 981 | - | 0.10 | 0.39 | 0.10 | 0.20 | 0.05 |
| Serratia marcescens IID 618 | - | 0.20 | 0.39 | 0.20 | 0.39 | 0.10 |
| S. marcescens GN 7577 | - | 1.56 | 6.25 | 3.13 | 3.13 | 0.78 |
| Pseudomonas aeruginosa V-1 | - | 0.78 | 1.56 | 1.56 | 1.56 | 0.39 |
| P. aeruginosa IFO 12689 | - | 1.56 | 3.13 | 3.13 | 3.13 | 0.78 |
| P. aeruginosa IID 1210 | - | 1.56 | 3.13 | 3.13 | 3.13 | 1.56 |
| Acinetobacter anitratus IID 876 | - | 0.10 | 0.78 | 0.05 | 0.10 | 0.05 |
| Alcaligenes faecalis 0104002 | - | 0.39 | 1.56 | 0.78 | 1.56 | 0.39 |
| Bacteroides fragilis GM 7000 | - | 0.78 | 25 | 1.56 | 1.56 | 0.20 |
| B. fragilis 0558 | - | 0.39 | 12.5 | 0.78 | 0.78 | 0.10 |
| B. fragilis 25285 | - | 0.39 | 25 | 0.78 | 0.78 | 0.10 |
| Fusobacterium varium KYA 8501 | - | 0.78 | 25 | 6.25 | 3.13 | 0.39 |
| Clostridium perfringens KYA 13123 | + | 0.39 | 1.56 | 0.39 | 0.20 | 0.10 |
| C. ramosum | + | 0.78 | 12.5 | 1.56 | 0.78 | 0.39 |
| C. difficile I-E | + | 1.56 | 12.5 | 6.25 | 3.13 | - |

TABLE 1-3

In vitro antibacterial activity (standard strain)

| Organism ($10^6$ cells/ml) | Gram | MIC (μg/ml) | | | | |
|---|---|---|---|---|---|---|
| | | Exp. 2 | Exp. 3 | Exp. 4 | Exp. 5 | Exp. 6 |
| Bacillus subtilis PCI 219 | + | 0.0125 | 0.0125 | 0.0125 | 0.0125 | 0.0125 |
| Staphylococcus aureus 209 P | + | 0.0125 | 0.0125 | 0.0125 | 0.0125 | 0.0125 |
| S. aureus Smith | + | 0.025 | 0.0125 | 0.0125 | 0.0125 | 0.0125 |
| S. aureus IID 670 (Terajima) | + | 0.025 | 0.0125 | 0.0125 | 0.025 | 0.025 |
| S. epidermidis IID 866 | + | 0.025 | 0.0125 | 0.0125 | 0.025 | 0.025 |
| Streptococcus pyogenes S-8 | + | 0.05 | 0.05 | 0.05 | 0.05 | 0.05 |
| S. pyogenes IID 692 | + | 0.05 | 0.05 | 0.05 | 0.10 | 0.05 |
| S. pneumoniae IID 552 | + | 0.05 | 0.025 | 0.05 | 0.05 | 0.05 |
| S. faecalis IID 682 | + | 0.05 | 0.05 | 0.10 | 0.05 | 0.05 |
| Escherichia coli NIHJ JC-2 | - | 0.0125 | 0.0125 | ≦0.0063 | 0.0125 | 0.0125 |
| E. coli ATCC 10536 | - | 0.025 | 0.0125 | 0.0125 | 0.025 | 0.025 |
| E. coli ML 4707 | - | 0.025 | 0.0125 | 0.025 | 0.025 | 0.025 |
| Proteus vulgaris IFO 3167 | - | 0.025 | 0.025 | 0.0125 | 0.025 | 0.025 |
| P. mirabilis IID 994 | - | 0.05 | 0.025 | 0.0125 | 0.025 | 0.025 |
| Morganella morganii IID 602 | - | 0.10 | 0.05 | 0.10 | 0.10 | 0.20 |
| Enterobacter cloacae IID 977 | - | 0.10 | 0.05 | 0.05 | 0.10 | 0.10 |
| Citrobacter freundii IID 976 | - | 0.05 | 0.025 | 0.025 | 0.05 | 0.05 |
| Klebsiella pneumoniae KY(GN)6445 | - | 0.05 | 0.025 | 0.025 | 0.05 | 0.05 |
| K. pneumoniae 1-220S | - | 0.10 | 0.05 | 0.05 | 0.10 | 0.10 |
| Salmonella entritidis IID 604 | - | 0.05 | 0.025 | 0.025 | 0.05 | 0.10 |
| Shigella sonnei IID 969 | - | 0.025 | 0.0125 | 0.0125 | 0.025 | 0.025 |
| Yersinia enterocolitica IID 981 | - | 0.10 | 0.05 | 0.05 | 0.10 | 0.10 |
| Serratia marcescens IID 618 | - | 0.10 | 0.05 | 0.10 | 0.10 | 0.10 |
| S. marcescens GN 7577 | - | 0.78 | 0.39 | 0.78 | 0.78 | 0.78 |
| Pseudomonas aeruginosa V-1 | - | 0.78 | 0.20 | 0.10 | 0.20 | 0.39 |
| P. aeruginosa IFO 12689 | - | 1.56 | 0.39 | 0.78 | 1.56 | 3.13 |
| P. aeruginosa IID 1210 | - | 1.56 | 0.39 | 0.78 | 1.56 | 1.56 |
| Acinetobacter anitratus IID 876 | - | 0.05 | 0.05 | 0.05 | 0.05 | 0.05 |
| Alcaligenes faecalis 0104002 | - | 0.39 | 0.20 | 0.20 | 0.39 | 0.39 |
| Bacteroides fragilis GM 7000 | - | 0.10 | 0.10 | 0.10 | 0.20 | 0.10 |
| B. fragilis 0558 | - | 0.10 | 0.05 | ≦0.05 | 0.10 | ≦0.05 |
| B. fragilis 25285 | - | 0.05 | 0.05 | ≦0.05 | 0.10 | ≦0.05 |
| Fusobacterium varium KYA 8501 | - | 0.39 | 0.39 | 0.39 | 0.39 | 0.39 |
| Clostridium perfringens KYA 13123 | + | 0.05 | 0.05 | 0.10 | 0.10 | 0.10 |
| C. ramosum | + | 0.20 | 0.39 | 0.39 | 0.39 | 0.39 |
| C. difficile I-E | + | - | - | - | - | - |

TABLE 1-4

In vitro antibacterial activity (standard strain)

| Organism ($10^6$ cells/ml) | Gram | MIC (μg/ml) | | | | |
|---|---|---|---|---|---|---|
| | | Exp. 9 | Ref. 12 | Ref. 13 | CFLX | NFLX |
| Bacillus subtilis PCI 219 | + | 0.025 | 0.05 | 0.05 | 0.05 | 0.10 |
| Staphylococcus aureus 209 P | + | 0.025 | 0.05 | 0.05 | 0.20 | 0.78 |
| S. aureus Smith | + | 0.025 | 0.05 | 0.10 | 0.39 | 1.56 |
| S. aureus IID 670 (Terajima) | + | 0.05 | 0.05 | 0.10 | 0.20 | 0.78 |
| S. epidermidis IID 866 | + | 0.05 | 0.05 | 0.10 | 0.20 | 0.39 |
| Streptococcus pyogenes S-8 | + | 0.05 | 0.10 | 0.20 | 0.78 | 1.56 |
| S. pyogenes IID 692 | + | 0.10 | 0.20 | 0.39 | 0.78 | 3.13 |
| S. pneumoniae IID 552 | + | 0.05 | 0.20 | 0.20 | 0.78 | 3.13 |
| S. faecalis IID 682 | + | 0.10 | 0.20 | 0.20 | 0.78 | 1.56 |
| Escherichia coli NIHJ JC-2 | - | 0.025 | 0.05 | 0.10 | 0.0125 | 0.025 |
| E. coli ATCC 10536 | - | 0.025 | 0.05 | 0.10 | 0.025 | 0.05 |
| E. coli ML 4707 | - | 0.05 | 0.05 | 0.10 | 0.0125 | 0.05 |
| Proteus vulgaris IFO 3167 | - | 0.05 | 0.05 | 0.05 | 0.0125 | 0.025 |
| P. mirabilis IID 994 | - | 0.05 | 0.10 | 0.20 | 0.025 | 0.05 |
| Morganella morganii IID 602 | - | 0.20 | 0.20 | 0.78 | 0.05 | 0.05 |
| Enterobacter cloacae IID 977 | - | 0.10 | 0.20 | 0.20 | 0.05 | 0.10 |
| Citrobacter freundii IID 976 | - | 0.05 | 0.05 | 0.20 | 0.025 | 0.05 |
| Klebsiella pneumoniae KY(GN)6445 | - | 0.05 | 0.05 | 0.20 | 0.025 | 0.05 |
| K. pneumoniae 1-220S | - | 0.10 | 0.20 | 0.39 | 0.05 | 0.20 |
| Salmonella entritidis IID 604 | - | 0.10 | 0.10 | 0.39 | 0.05 | 0.10 |
| Shigella sonnei IID 969 | - | 0.05 | 0.05 | 0.10 | 0.0125 | 0.05 |
| Yersinia enterocolitica IID 981 | - | 0.10 | 0.10 | 0.39 | 0.05 | 0.10 |
| Serratia marcescens IID 618 | - | 0.10 | 0.20 | 0.39 | 0.05 | 0.05 |
| S. marcescens GN 7577 | - | 1.56 | 1.56 | 6.25 | 0.78 | 1.56 |
| Pseudomonas aeruginosa V-1 | - | 0.39 | 0.39 | 6.25 | 0.39 | 0.78 |
| P. aeruginosa IFO 12689 | - | 3.13 | 3.13 | 6.25 | 0.39 | 0.78 |
| P. aeruginosa IID 1210 | - | 1.56 | 3.13 | 12.5 | 1.56 | 3.13 |
| Acinetobacter anitratus IID 876 | - | 0.05 | 0.05 | 0.78 | 0.39 | 3.13 |
| Alcaligenes faecalis 0104002 | - | 0.39 | 0.78 | 12.5 | 0.39 | 3.13 |
| Bacteroides fragilis GM 7000 | - | 0.10 | 0.39 | 12.5 | 3.13 | 25 |
| B. fragilis 0558 | - | ≦0.05 | 0.20 | 6.25 | 3.13 | 25 |
| B. fragilis 25285 | - | ≦0.05 | 0.20 | 6.25 | 3.13 | 25 |
| Fusobacterium varium KYA 8501 | - | 0.78 | 3.13 | 6.25 | 12.5 | 100 |
| Clostridium perfringens KYA 13123 | + | 0.10 | 0.20 | 1.56 | 0.39 | 1.56 |
| C. ramosum | + | 0.39 | 0.78 | 6.25 | 3.13 | 50 |
| C. difficile I-E | + | - | - | - | 12.5 | 50 |

Ref. 13: 1-Ethyl-7-(3-ethylaminomethyl-1-pyrrolidinyl)-6,8-difluoro-1,4-dihydro-4-oxo-3-quinolinecarboxylic acid

CFLX: Ciprofloxacin

NFLX: Norfloxacin

TABLE 2-1

In vitro antibacterial activity (1) (clinical isolates)

| Organism ($10^6$ cells/ml) | Gram | MIC (μg/ml) | | | | | | | | | | | | | |
|---|---|---|---|---|---|---|---|---|---|---|---|---|---|---|---|
| | | Ref. 1 | Ref. 2 | Ref. 4 | Ref. 6 | Ref. 7 | Ref. 8 | Ref. 9 | Ref. 10 | Ref. 11 | Exp. 1 | Exp. 2 | Exp. 3 | Exp. 4 | Exp. 5 |
| S. pneumoniae 15 | | 0.05 | 0.05 | 0.05 | 0.20 | 0.39 | 0.05 | 0.39 | 0.39 | 0.20 | 0.05 | 0.025 | 0.025 | 0.05 | 0.05 |
| S. pneumoniae 24 | | 0.05 | 0.05 | 0.05 | - | - | 0.05 | - | - | - | 0.05 | 0.05 | 0.05 | - | - |
| S. pneumoniae 28 | | 0.05 | 0.05 | 0.05 | - | - | 0.05 | - | - | - | 0.05 | 0.05 | 0.05 | - | - |
| S. pneumoniae 2054 | + | 0.05 | 0.10 | 0.05 | 0.39 | 0.39 | 0.05 | 0.78 | 0.78 | 0.20 | 0.05 | 0.05 | 0.05 | 0.10 | 0.05 |
| S. pneumoniae 2950 | | 0.05 | 0.10 | 0.05 | 0.20 | 0.39 | 0.10 | 0.78 | 0.39 | 0.20 | - | - | - | 0.05 | 0.05 |
| S. pneumoniae 3227 | | 0.05 | 0.10 | 0.05 | 0.20 | 0.39 | - | 0.39 | 0.20 | 0.39 | 0.05 | 0.05 | 0.05 | 0.05 | 0.05 |
| S. pyogenes 3130 | | 0.05 | 0.05 | 0.05 | 0.05 | 0.20 | 0.05 | 0.10 | 0.20 | 0.20 | 0.05 | 0.05 | 0.025 | 0.05 | 0.05 |
| S. pyogenes 3102 | | 0.05 | 0.05 | 0.05 | 0.05 | 0.20 | 0.05 | 0.10 | 0.20 | 0.20 | 0.05 | 0.05 | 0.025 | 0.05 | 0.05 |
| S. pyogenes 3107 | + | 0.05 | 0.05 | 0.05 | 0.05 | 0.20 | 0.05 | 0.10 | 0.20 | 0.20 | 0.05 | 0.05 | 0.025 | 0.05 | 0.05 |
| S. pyogenes 4340 | | 0.05 | 0.10 | 0.05 | 0.05 | 0.20 | 0.05 | 0.20 | 0.20 | 0.20 | 0.05 | 0.05 | 0.05 | 0.05 | 0.05 |
| S. pyogenes 4372 | | 0.05 | 0.10 | 0.05 | 0.10 | 0.20 | 0.05 | 0.20 | 0.39 | 0.20 | 0.05 | 0.05 | 0.05 | 0.05 | 0.05 |
| S. agalactiae 4394 | | 0.10 | 0.20 | 0.10 | 0.39 | 0.39 | 0.10 | 0.78 | 0.78 | 0.39 | 0.10 | 0.10 | 0.10 | 0.20 | 0.10 |
| S. agalactiae 4049 | | 0.05 | 0.10 | 0.05 | 0.39 | 0.78 | 0.20 | 0.78 | 0.78 | 0.39 | 0.05 | 0.05 | 0.05 | 0.05 | 0.05 |
| S. agalactiae 4342 | | 0.05 | 0.10 | 0.05 | 0.20 | 0.39 | 0.20 | 0.39 | 0.20 | 0.20 | 0.05 | 0.05 | 0.05 | 0.10 | 0.05 |
| S. agalactiae 4470 | + | 0.05 | 0.10 | 0.05 | 0.20 | 0.39 | - | 0.39 | 0.39 | 0.20 | 0.05 | 0.05 | 0.05 | 0.05 | 0.05 |
| S. agalactiae 4368 | | 0.05 | 0.10 | 0.05 | 0.20 | 0.39 | 0.10 | 0.20 | 0.39 | 0.20 | 0.05 | 0.025 | 0.05 | 0.05 | 0.05 |
| S. agalactiae 4468 | | 0.05 | 0.10 | 0.05 | 0.20 | 0.39 | 0.10 | 0.39 | 0.39 | 0.20 | 0.05 | 0.05 | 0.05 | 0.05 | 0.05 |
| S. faecalis 49 | | 0.10 | 0.20 | 0.10 | 0.39 | 0.78 | 0.10 | 0.39 | 0.39 | 0.20 | 0.05 | 0.05 | 0.05 | 0.20 | 0.10 |
| S. faecalis 214 | | 0.10 | 0.20 | 0.10 | 0.39 | 0.78 | 0.05 | 0.39 | 0.39 | 0.39 | 0.10 | 0.10 | 0.05 | 0.20 | 0.10 |
| S. faecalis 401 | + | 0.10 | 0.20 | 0.10 | 0.39 | 0.78 | 0.10 | 0.78 | 0.78 | 0.39 | 0.10 | 0.10 | 0.05 | 0.20 | 0.10 |
| S. faecalis 402 | | 0.10 | 0.20 | 0.10 | 0.39 | 0.78 | 0.20 | 0.78 | 0.39 | 0.39 | 0.10 | 0.10 | 0.05 | 0.20 | 0.10 |

TABLE 2—2
In vitro antibacterial activity (2)
(clinical isolates)

| Organism ($10^6$ cells/ml) | Gram | MIC (µg/ml) | | | | | |
|---|---|---|---|---|---|---|---|
| | | Exp. 6 | Exp. 9 | Ref. 12 | Ref. 13 | CFLX | NFLX |
| S. pneumoniae 15 | + | 0.05 | 0.05 | 0.20 | 0.20 | 0.78 | 3.13 |
| S. pneumoniae 24 | | — | — | — | — | 1.56 | 12.5 |
| S. pneumoniae 28 | | — | — | — | — | 0.78 | 6.25 |
| S. pneumoniae 2054 | | 0.05 | 0.10 | 0.20 | 0.39 | 1.56 | 6.25 |
| S. pneumoniae 2950 | | 0.05 | 0.05 | 0.10 | 0.39 | 1.56 | 12.5 |
| S. pneumoniae 3227 | | 0.05 | 0.10 | 0.20 | 0.39 | 1.56 | 12.5 |
| S. pyogenes 3130 | + | 0.05 | 0.05 | 0.10 | 0.20 | 0.39 | 1.56 |
| S. pyogenes 3102 | | 0.05 | 0.05 | 0.10 | 0.20 | 0.39 | 1.56 |
| S. pyogenes 3107 | | 0.05 | 0.05 | 0.10 | 0.10 | 0.39 | 1.56 |
| S..pyogenes 4340 | | 0.05 | 0.05 | 0.10 | 0.20 | 0.78 | 1.56 |
| S. pyogenes 4372 | | 0.05 | 0.10 | 0.20 | 0.20 | 0.78 | 1.56 |
| S. agalactiae 4394 | + | 0.10 | 0.20 | 0.20 | 0.78 | 3.13 | 6.25 |
| S. agalactiae 4049 | | 0.05 | 0.10 | 0.20 | 0.39 | 0.78 | 6.25 |
| S. agalactiae 4342 | | 0.05 | 0.10 | 0.20 | 0.39 | 0.78 | 6.25 |
| S. agalactiae 4470 | | 0.05 | 0.10 | 0.20 | 0.39 | 0.78 | 6.25 |
| S. agalactiae 4368 | | 0.05 | 0.05 | 0.20 | 0.20 | 0.78 | 3.13 |
| S. agalactiae 4468 | | 0.05 | 0.10 | 0.20 | 0.39 | 0.78 | 3.13 |
| S. faecalis 49 | + | 0.10 | 0.10 | 0.20 | 0.39 | 1.56 | 3.13 |
| S. faecalis 214 | | 0.10 | 0.10 | 0.20 | 0.39 | 1.56 | 3.13 |
| S. faecalis 401 | | 0.10 | 0.10 | 0.20 | 0.39 | 1.56 | 6.25 |
| S. faecalis 402 | | 0.10 | 0.10 | 0.20 | 0.78 | 1.56 | 6.25 |

Experiment 2

In vivo antibacterial activity against systemic infection in mice (ICR-S)

The infections were produced by intraperitoneal injection of a suspension of the bacterial culture corresponding to the germ studied. The products were administered orally at an hour after the infection. The 50% effective dose ($ED_{50}$) which protects 50% of the animals from death caused by the infection was determined from the relation between dosage and survival rate.

The efficacy of the compounds of the invention is shown in Table 3 together with that of the known compound.

The present compounds are the most effective against gram-positive bacterial infections.

TABLE 3
In vivo antibacterial activity against systemic infection in mice (ICR-S)

| Compound | ED50 (mg/kg) | | |
|---|---|---|---|
| | S. aureus Smith[a)] | S. pneumoniae S-4288[b)] | E. coli ML4707[c)] |
| Reference example 2 | 0.9 | 4.6 | 0.8 |
| " " 4 | 1.8 | 13.8 | 3.0 |
| " " 8 | 1.1 | 16.1 | 1.9 |
| Example 1 | 0.5 | 6.5 | 1.9 |
| " 2 | 0.6 | 2.0 | 0.7 |
| " 3 | — | 10.0 | 5.4 |
| " 4 | — | — | 7.1 |
| " 5 | — | — | 1.9 |
| " 6 | — | — | 0.9 |
| " 9 | — | — | 2.1 |
| Reference example 12 | — | — | 1.2 |
| Ref. 13 | 5.0 | 62.2 | >10 |
| CFLX | >25 | >100 | 1.8 |

a) Challenge dose: $6.0 \times 10^5$ cfu/mouse
b) Challenge dose: $7.5 \times 10^4 \sim 4.8 \times 10^6$ cfu/mouse
c) Challenge dose: $2.7 \times 10^6 \sim 8.8 \times 10^6$ cfu/mouse.

Experiment 3

In vitro antibacterial activity against Mycoplasma

The antimicrobial activity of the present compounds has been investigated in vitro on M. pneumoniae Mac. Table 4 gives the minimum inhibitory concentrations (MIC) of the present compounds together with those of the reference compounds. The present compounds are the most active.

TABLE 4
In vitro antibacterial activity against Mycoplasma (M. pneumoniae Mac.)

| Compound | MIC* (µg/ml) |
|---|---|
| Example 1 | 0.05 |
| Example 2 | 0.05 |
| Ref. 13 | 0.39 |
| CFLX | 0.78 |
| NFLX | 3.13 |
| TC | 0.78 |

TC: Tetracycline

*The MIC was defined as the concentration of agent at which there was no visible growth after incubation for 5 days.

Experiment 4

Absorption, distribution and excretion

The blood, organs, bile and urine of the fasted rats, which had received the dose of 10 mg/kg of the test compound by oral administration, were sampled over a period of time and the proportion of active compound was determined bacteriologically by the cup method using E. coli NIHJ JC-2. The results are shown in Tables 5 and 6. All those results make it possible to guarantee a greater therapeutic action of the present compound than the reference compound because of higher concentration of active compound in comparison with the reference compound.

TABLE 5
Concentration in organs (µg/ml or g)

| Organ | Compound | Time after administration (hr) | | | | | |
|---|---|---|---|---|---|---|---|
| | | 1/2 | 1 | 2 | 4 | 6 | 8 |
| Serum | Example 2 | 1.72 ± 0.37 | 1.12 ± 0.30 | 0.57 ± 0.08 | 0.23 ± 0.04 | 0.10 ± 0.01 | 0.14 ± 0.08 |
| | CFLX | 0.47 ± 0.28 | 0.38 ± 0.11 | 0.25 ± 0.05 | 0.09 ± 0.03 | 0.05 ± 0.01 | 0.03 ± 0.01 |
| Lung | Example 2 | 3.17 ± 0.71 | 2.66 ± 0.42 | 1.54 ± 0.21 | 0.70 ± 0.08 | 0.42 ± 0.05 | 0.45 ± 0.15 |
| | CFLX | 0.86 ± 0.58 | 0.60 ± 0.19 | 0.29 ± 0.02 | 0.14 ± 0.02 | 0.07 ± 0.03 | 0.06 ± 0.01 |
| Liver | Example 2 | 7.78 ± 0.91 | 5.71 ± 0.63 | 3.14 ± 0.36 | 1.34 ± 0.12 | 0.86 ± 0.15 | 1.13 ± 0.52 |
| | CFLX | 5.17 ± 2.53 | 3.28 ± 0.98 | 1.49 ± 0.37 | 0.39 ± 0.05 | 0.21 ± 0.11 | 0.15 ± 0.05 |
| Kidney | Example 2 | 5.63 ± 0.64 | 4.70 ± 0.60 | 2.81 ± 0.25 | 1.47 ± 0.25 | 0.84 ± 0.09 | 0.89 ± 0.10 |
| | CFLX | 3.58 ± 2.61 | 2.05 ± 0.64 | 1.12 ± 0.34 | 0.21 ± 0.02 | 0.10 ± 0.04 | 0.06 ± 0.02 |

Mean ± S.D. (n = 5)

TABLE 6
Urinary and bile excretion

| Sample | Compound | Concentration (µg/ml) | | | Recovery (%) | | |
|---|---|---|---|---|---|---|---|
| | | 0 ~ 3 | 3 ~ 6 | 6 ~ 24 hr | 0 ~ 3 | 0 ~ 6 | 0 ~ 24 hr |
| Urine | Example 2 | 36.1 ± 9.2 | 39.4 ± 13.3 | 7.6 ± 3.6 | 2.7 ± 2.1 | 4.8 ± 1.8 | 7.4 ± 1.5 |
| | CFLX | 101 ± 36 | 57 ± 19 | 12 ± 6 | 3.9 ± 2.9 | 6.6 ± 2.2 | 8.9 ± 1.8 |
| Bile | Example 2 | 36.4 ± 9.9 | 19.7 ± 8.0 | 11.3 ± 8.3 | 4.9 ± 1.5 | 6.9 ± 1.4 | 12.9 ± 4.9 |
| | CFLX | 11.1 ± 4.4 | 5.9 ± 2.6 | 1.5 ± 0.6 | 1.3 ± 0.7 | 1.9 ± 0.5 | 2.5 ± 0.8 |

Mean ± S.D. (n = 5).

**Claims**

1. A compound of the formula (I)

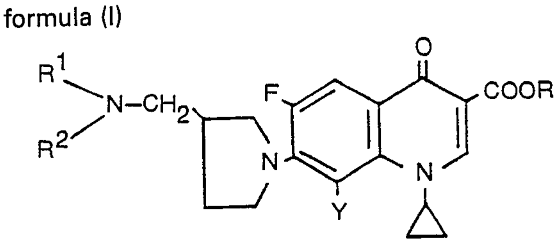

(I)

wherein R is a hydrogen atom or an ethyl group and $R^1$ and $R^2$ are each independently a hydrogen atom, a

methyl, an ethyl, a n-propyl or an i-propyl group and Y is a chlorine atom or a bromine atom; the hydrates or the pharmaceutically acceptable acid addition or alkali salts thereof.

2. A process for the preparation of a compound of the formula (I) according to claim 1 comprising condensing a compound of the formula (II)

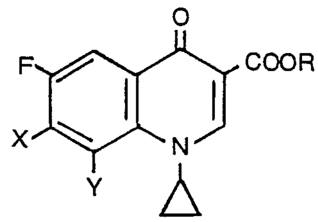

(II)

wherein R is a hydrogen atom or an ethyl group, X is a halogen atom and Y is a chlorine atom or a bromine atom; with a secondary amine of the formula (III)

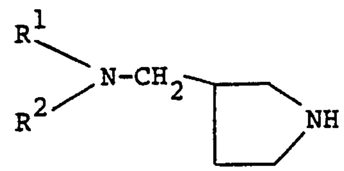

(III)

wherein $R^1$ and $R^2$ are each independently a hydrogen atom, a methyl, an ethyl or a n-propyl or an i-propyl group.

3. A process for the preparation of a compound of the formula (I) according to claim 1 wherein R is a hydrogen atom, comprising hydrolyzing a compound of the formula (IV)

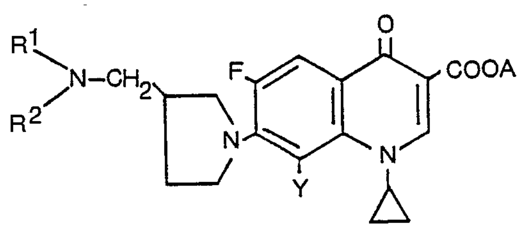

(IV)

wherein $R^1$ and $R^2$ are each independently a hydrogen atom, a methyl, an ethyl or a n-propoyl or an i-propyl group, Y is a chlorine atom or a bromine atom and A is an ethyl group.

4. An antibacterial pharmaceutical composition comprising at least one compound according to claim 1 and an inert pharmaceutical acceptable carrier.

**Patentansprüche**

1. Verbindung der Formel (I)

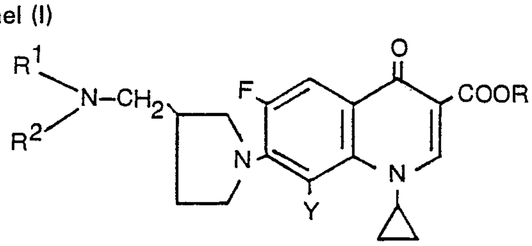

(I)

worin R ein Wasserstoffatom oder eine Ethylgruppe und $R^1$ und $R^2$ jeweils voneinander unabhängig ein Wasserstoffatom, eine Methyl-, eine Ethyl-, eine n-Propyl- oder eine i-Propylgruppe und Y ein Chlor- oder ein Bromatom bedeuten; die Hydrate oder die pharmazeutisch annehmbaren Säure- oder Alkaliadditionssalze davon.

2. Verfahren zur Herstellung einer Verbindung der Formel (I) nach Anspruch 1, dadurch gekennzeichnet, daß man eine Verbindung der Formel (II)

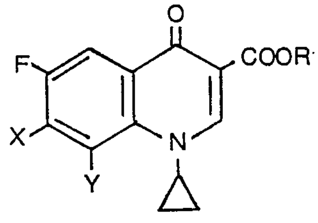

(II)

worin R ein Wasserstoffatom oder eine Ethylgruppe, X ein Halogenatom und Y ein Chlor- oder ein Bromatom bedeuten; mit einem sekundären Amin der Formel (III)

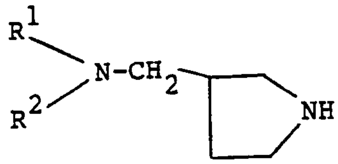

(III)

worin $R^1$ und $R^2$ jeweils voneinander unabhängig ein Wasserstoffatom, eine Methyl-, eine Ethyl- oder eine n-Propyl- oder eine i-Propylgruppe bedeuten, kondensiert.

3. Verfahren zur Herstellung einer Verbindung der Formel (I) nach Anspruch 1, worin R ein Wasserstoffatom bedeutet, dadurch gekennzeichnet, daß man eine Verbindung der Formel (IV)

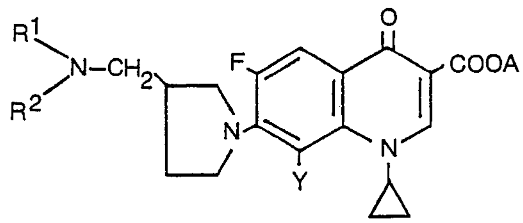

(IV)

worin $R^1$ und $R^2$ jeweils voneinander unabhängig ein Wasserstoffatom, eine Methyl-, eine Ethyl- oder eine n-Propyl- oder i-Propylgruppe, Y ein Chlor- oder ein Bromatom und A eine Ethylgruppe bedeuten, hydrolisiert.

4. Antibakterielle pharmazeutische Zusammensetzung, enthaltend mindestens eine Verbindung nach Anspruch 1 und einen inerten pharmazeutisch annehmbaren Trägerstoff.

**Revendications**

1. Composé de formule (I)

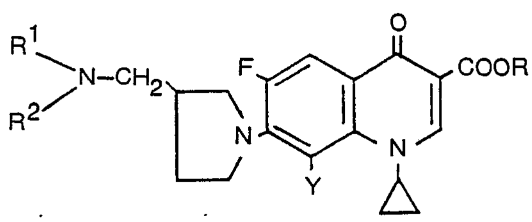

(I)

dans laquelle R est un atome d'hydrogène ou un groupe éthyle et $R^1$ et $R^2$ sont chacun, indépendamment, un atome d'hydrogène ou un groupe méthyle, éthyle, n-propyle ou isopropyle, et Y est un atome de chlore ou de brome; les hydrates ou les sels alcalins ou d'addition avec des acides pharmaceutiquement acceptables de ceux-ci.

2. Procédé pour la préparation d'un composé de formule (I) selon la revendication 1, comprenant la condensation d'un composé de formule (II)

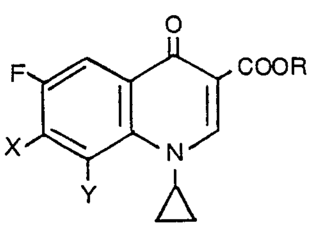

(II)

dans laquelle R est un atome d'hydrogène ou le groupe éthyle, X est un atome d'halogène et Y est un atome de chlore ou de brome; avec une amine secondaire de formule (III)

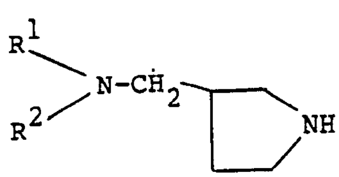

(III)

dans laquelle $R^1$ et $R^2$ sont chacun, indépendamment, un atome d'hydrogène ou un groupe méthyle, éthyle, n-propyle ou isopropyle.

3. Procédé pour la préparation d'un composé de formule (I) selon la revendication 1, dans lequel R est un atome d'hydrogène, comprenant l'hydrolyse d'un composé de formule (IV)

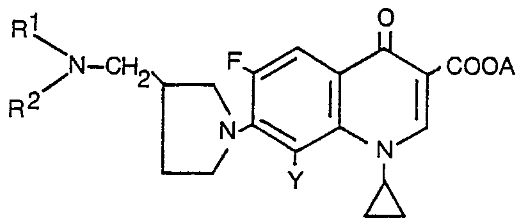

(IV)

dans laquelle $R^1$ et $R^2$ sont chacun, independamment, un atome d'hydrogène ou un groupe méthyle, éthyle, n-propyle ou isopropyle, Y est un atome de chlore ou de brome, et A est le groupe éthyle.

4. Composition pharamaceutique antibactérienne comprenant au moins un composé selon la revendication 1 et un véhicule inerte pharmaceutiquement acceptable.